# FASCICULE DE BREVET EUROPEEN

(11) **EP 1 429 673 B1**
(45) Date de publication et mention de la délivrance du brevet: **28.12.2005**
(21) Numéro de dépôt: 02798757.7
(22) Date de dépôt: 03.09.2002
(51) Int. Cl.: A61B 17/70

(54) **SYSTEME D'OSTEOSYNTHESE RACHIDIENNE COMPRENANT UN PATIN D'APPUI**
WIRBELOSTEOSYNTHESE-SYSTEM MIT EINEM STÜTZPOLSTER
SPINAL OSTEOSYNTHESIS SYSTEM COMPRISING A SUPPORT PAD

(30) Priorité: 03.09.2001 FR 0111363
(43) Date de publication de la demande: 23.06.2004
(73) Titulaire: Stryker Spine, 33610 Cestas (FR)
(72) Inventeur: VIENNEY, Cécile, F-33000 Bordeaux (FR); DE CONINCK, Cédric, F-33610 Cestas Gazinet (FR)
(74) Mandataire: Le Forestier, Eric
(86) Numéro de dépôt international: PCT/FR2002/002998
(87) Numéro de publication internationale: WO 2003/024343

(56) Documents cités:
- WO-A-01/08574
- WO-A-01/52758
- DE-U- 9 419 900
- FR-A- 2 778 089
- FR-A- 2 810 533
- US-A- 5 474 555

## Description

L'invention concerne des moyens de serrage destinés à des systèmes d'ostéosynthèse rachidienne.

Le document FR 2 680 461 présente un dispositif comprenant un organe d'ancrage osseux de type vis pédiculaire comprenant une tête en « U » apte à recevoir, d'une part, un verrou et, d'autre part, un anneau entourant les branches formant le « U ». De plus, l'anneau comprend une barre diamétrale apte à venir entre les branches de l'ouverture en « U » et apte à venir s'intercaler entre le verrou et une tige de liaison reçue entre les branches du « U », lors du serrage du montage. Ce dispositif présente des inconvénients en termes de volume et de complexité :
- l'anneau est volumineux et s'étend en saillie de la surface externe de la tête de l'organe d'ancrage alors qu'un système d'ostéosynthèse doit être aujourd'hui invasif au minimum.
- l'anneau étant monté glissant sur les branches du « U », lors du serrage du verrou, la forme des filets oblige les branches à s'écarter bloquant la descente de l'anneau, ainsi que la déformation de la branche diamétrale. Il en résulte un mauvais appui de la branche diamétrale sur la tige de liaison conduisant à un risque.élevé de desserrage du système d'ostéosynthèse préjudiciable pour le patient ;
- le verrou et l'anneau sont indépendants l'un de l'autre rendant le montage complexe et fastidieux.

Le document WO 01/52758 décrit un dispositif selon le préambule de la revendication 1.

Un but de l'invention est de fournir des moyens de serrage pour un système d'ostéosynthèse rachidienne permettant d'assurer un effort axial de blocage répétable et suffisant pour un couple de serrage minime, tout en étant simple et peu volumineux.

Pour cela, on prévoit, selon l'invention, un système d'ostéosynthèse selon la revendication 1.

Ainsi, les moyens d'interposition restent invasifs au minimum tout en permettant d'assurer un effort axial de blocage répétable et suffisant pour un couple de serrage minime.

Avantageusement, le système d'ostéosynthèse rachidienne présente au moins l'une des caractéristiques suivantes :
- les moyens d'interposition sont agencés de sorte qu'ils soient aptes à venir en appui sur l'organe de liaison selon au moins une génératrice de l'organe de liaison ;
- les moyens d'interposition sont agencés de sorte qu'ils soient aptes à venir en appui sur l'organe de liaison selon deux génératrices parallèles de l'organe de liaison ;
- les moyens d'interposition présentent une surface plane perpendiculaire à un axe de rotation du verrou apte à venir en appui avec le verrou ;
- les moyens d'interposition présentent une face concave et une face délimitant au moins une arête apte à venir en appui contre l'organe de liaison;
- la face concave est une portion de face cylindrique dont un diamètre moyen est inférieur à une dimension de l'organe de liaison ;
- la face concave est délimitée par au moins deux arêtes aptes à réaliser l'appui contre l'organe de liaison ;
- le système présente des moyens de retenue des moyens d'interposition directement sur le verrou ;
- les moyens d'interposition sont aptes à être montés à rotation sur le verrou ;
- les moyens de retenue comportent une lèvre ;
- les moyens de retenue comprennent au moins un ergot apte à venir en appui sur la lèvre ;
- le verrou comporte la lèvre et les moyens d'interposition comprennent l'ergot ;
- le verrou comporte l'ergot et les moyens d'interposition comprennent la lèvre ;
- les moyens de retenue comprennent un filetage apte à être retenu prisonnier par la lèvre ;
- la lèvre fait partie d'une gorge ;
- les moyens d'interposition présentent une épaisseur e entre leurs faces supérieure et inférieure, apte à les rendre sensiblement indéformables lors d'un serrage du système.

D'autres caractéristiques et avantages de l'invention apparaîtront lors de la description suivante d'un mode préféré de réalisation. Aux dessins annexés :
la figure 1 est une vue partielle en trois dimensions d'un système d'ostéosynthèse selon un mode préféré de réalisation de l'invention ;
la figure 2 est une vue de côté du système d'ostéosynthèse de la figure 1 ;

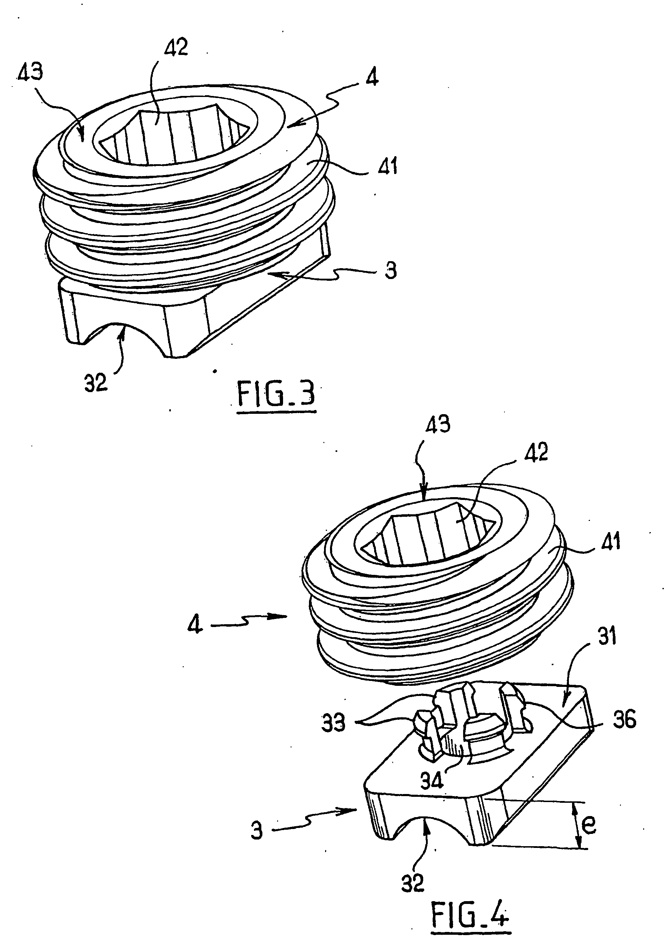

la figure 3 est une vue en trois dimensions du verrou et du patin du système d'ostéosynthèse de la figure 1 ;
la figure 4 est une vue éclatée en trois dimensions des pièces de la figure 3 ;
la figure 5 est une vue en trois dimensions et en coupe des pièces de la figure 3 ;
la figure 6 est une vue en trois dimensions du verrou de la figure 3 ; et
la figure 7 est une vue en trois dimensions du patin de la figure 3.

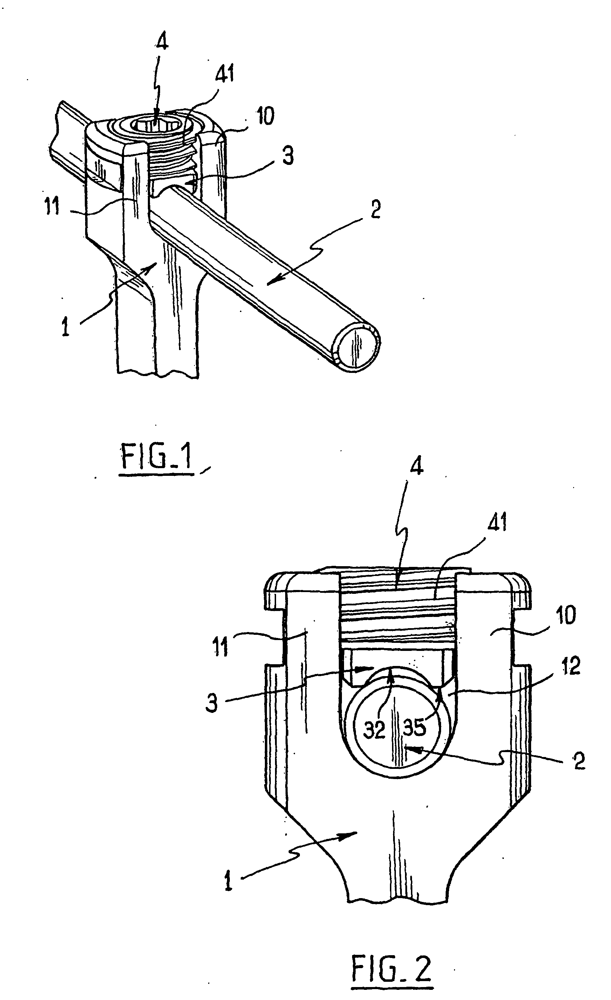

En référence aux figures 1 et 2, le système d'ostéosynthèse comprend plusieurs sous-ensembles comprenant chacun un organe d'ancrage 1 présentant une tête apte à recevoir un organe de liaison 2 ainsi qu'un verrou 4 et un patin de serrage 3. L'un de ces sous-ensembles est illustré à la figure 1.

L'organe d'ancrage 1 présente une tête comportant deux bras 11 et 12 s'étendant en regard l'un de l'autre pour former une ouverture en « U » 12. L'organe de liaison 2, ici une tige de liaison, est reçu dans l'ouverture en « U » 12 de l'organe d'ancrage 1. Les faces intérieures en regard l'une de l'autre des branches 11 et 12 de la tête de l'organe d'ancrage comportent un filetage apte à coopérer avec un filetage 41 du verrou 4. Le patin 3 peut être positionné entre l'organe d'ancrage 2 et le verrou 4.

Le verrou 4 a une forme générale de cylindre. Sa face extérieure latérale présente un filetage 41 apte à venir coopérer avec le filetage complémentaire de l'organe d'ancrage 1. Le verrou 4 présente une face supérieure 43 perpendiculaire à son axe de révolution (non représenté). A partir de cette face 43, l'organe 4 présente un orifice circulaire central 47 se terminant par une lèvre 46 s'étendant en saillie interne suivant une direction radiale à l'axe. Dans la majeure partie de l'orifice 47, des moyens de mise en oeuvre 42 du verrou 4 sont réalisés et s'étendent à partir de la face supérieure 43 et ce, sur la majeure partie de l'orifice 47. A partir du bord 46 de l'orifice 47, un trou traversant 44 de section circulaire est réalisé dans le verrou 4 jusqu'à une face inférieure 45 parallèle à la face supérieure 43. Le diamètre du trou 44 est inférieur au diamètre de l'orifice 47, ce qui permet d'obtenir la lèvre 46 entre le trou 44 et l'orifice 47.

Le patin 3 est de forme générale parallélépipédique rectangle. Il présente une largeur telle qu'il puisse s'insérer entre les deux branches 10 et 11 de l'organe d'ancrage formant l'ouverture 12. Le patin de serrage 3 présente une longueur telle que, une fois inséré dans l'ouverture en « U » 12, il ne dépasse pas de part et d'autre de la tête de l'organe d'ancrage 1 suivant la direction longitudinale de l'organe de liaison 2. De manière préférentielle, la longueur du patin de serrage 3 est sensiblement égale au plus grand diamètre extérieur du verrou 4.

Le patin de serrage 3 présente une face supérieure plane 31 au centre de laquelle peut être réalisé un orifice traversant 34 sur toute l'épaisseur du patin de serrage 3. Autour de cet orifice, sont disposés, uniformément répartis autour de cet orifice 34, plusieurs ergots 33 s'étendant en saillie à partir de la face 31 du patin de serrage 3. Ici, les ergots sont au nombre de quatre. Chacun des ergots présente à son extrémité libre sur sa face extérieure une dent 36. D'autre part, chacun des ergots 33 est déformable de manière élastique en flexion radiale par rapport à l'axe de l'orifice. Le patin de serrage 3 présente sur sa face inférieure, opposée à la face supérieure 31, une face concave 32 profilée de section circulaire et s'étendant sur toute la longueur du patin de serrage 3. De part et d'autre de cette face concave, le patin de serrage 3 présente sur toute sa longueur, une face plane 35 perpendiculaire à un axe de l'orifice 34 et parallèle à la face supérieure 31. L'intersection de cette face plane 35 avec la face concave 32 détermine une arête qui est apte à venir en appui, lors du serrage, avec la surface de l'organe de liaison 2, comme nous le verrons plus loin. Dans des variantes de réalisation, la face 35 peut être principalement concave ou convexe.

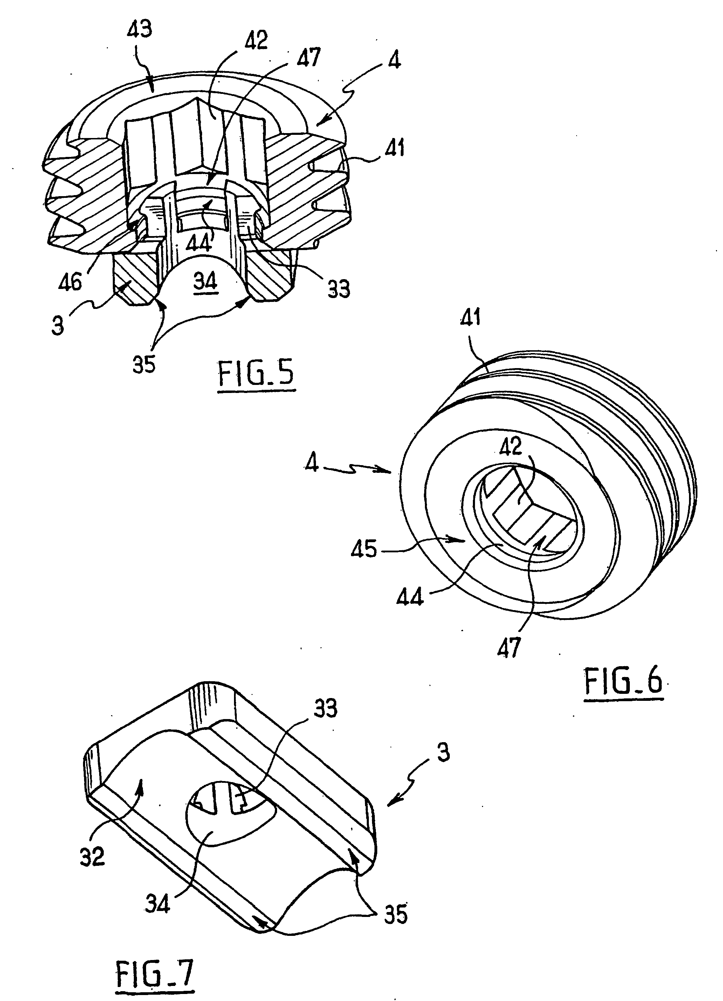

Le patin de serrage 3 est apte à être monté sur le verrou 4, comme cela est illustré à la figure 5. Pour cela, les ergots 33 du patin de serrage 3 sont insérés dans le trou 44 du verrou 4. Lors de cette insertion, les ergots 33 se déforment vers l'intérieur de l'orifice 34, et ce de manière élastique. Une fois que les dents 36 des ergots 33 débouchent dans l'orifice 47, les ergots 33, du fait de leur élasticité, reviennent à leur position initiale mettant les dents 36 de chacun des ergots 33 en contact avec la saillie interne radiale 46. Ainsi, le patin de serrage 3 est monté de façon imperdable sur le verrou 4, tout en laissant le verrou 4 libre de rotation par rapport au patin de serrage 3.

Lors de l'utilisation, le chirurgien, une fois qu'il a positionné l'organe de liaison 2 au sein de l'ouverture en « U » de l'organe d'ancrage 1, introduit l'assemblage formé du patin de serrage et du verrou 4 entre les branches 10 et 11 de l'organe d'ancrage, le patin de serrage 3 étant dirigé vers l'organe de liaison 2. Lorsque le chirurgien met en rotation le verrou 4 pour que ce dernier descende au sein de l'ouverture en « U », le patin de serrage glisse le long des branches 10 et 11 de l'organe d'ancrage 1 poussé par le verrou 4 jusqu'à venir en contact avec l'organe de liaison 2. Le chirurgien, réalise alors le serrage de son assemblage, le blocage de l'organe de liaison 2 s'effectuant par contact de celui-ci avec le patin de serrage via les arêtes déterminées par l'intersection de la face concave 32 et des faces planes 35 de ce dernier. On réalise ainsi un contact linéique selon deux génératrices de l'organe de liaison 2. Cela est rendu possible par le fait que le rayon moyen de la section de la face concave 32 du patin de serrage 3 est inférieur au rayon de la tige formant l'organe de liaison 2. De plus, sous la pression de serrage, l'arête va mâter la surface de l'organe de liaison assurant une sécurité supplémentaire contre le desserrage de l'assemblage.

Bien entendu, on pourra apporter à l'invention de nombreuses modifications, sans pour autant sortir du cadre ce celle-ci. Par exemple :
- le rebord peut être remplacé par une gorge ;
- les ergots 33 pourront être remplacés par un filetage qui viendra coopérer avec un filetage équivalent aménagé dans le trou 44, le filetage du patin de serrage surmontant une gorge apte à recevoir le filetage réalisé sur l'orifice 44. Ce dernier sera alors prisonnier lors de l'assemblage du patin de serrage avec le verrou dans cette gorge, le filetage prisonnier pourra être sur le patin de serrage et la gorge pourra être sur le verrou.
- la face concave 32 pourra avoir un rayon sensiblement équivalent à celui de l'organe de liaison 2, mais présentera alors une multitude d'arêtes parallèles les unes aux autres, uniformément réparties sur toute la surface de cette face, ces arêtes venant en prise avec la surface de l'organe de liaison lors du serrage.

## Revendications

1. Système d'ostéosynthèse rachidienne comprenant un organe d'ancrage (1), un organe de liaison (2), un verrou (4), et un organe d'interposition (3) apte à être placé en position de montage entre le verrou et l'organe de liaison lorsque ceux-ci sont reçus dans l'organe d'ancrage et agencés pour ne pas dépasser de l'organe d'ancrage en position de montage, l'organe de liaison est une tige de section circulaire, **caractérisé en ce que** l'organe d'interposition (3) présente au moins une arête, et **en ce que** l'organe d'interposition est aptes à réaliser un appui sur l'organe de liaison seulement selon la ou les arêtes.

2. Système selon la revendication 1, **caractérisé en ce que** l'organe d'interposition (3) est agencé pour venir en appui sur l'organe de liaison selon au moins une génératrice de l'organe de liaison.

3. Système selon la revendication 1 ou 2, **caractérisé en ce que** l'organe d'interposition (3) est agencé pour venir en appui sur l'organe de liaison selon deux génératrices parallèles de l'organe de liaison.

4. Système selon une des revendications 1 à 3 **caractérisé en ce que** l'organe d'interposition (3) présente une surface plane (31) perpendiculaire à un axe de rotation du verrou et apte à venir en appui contre le verrou.

5. Système selon l'une des revendications 1 à 4, **caractérisé en ce que** l'organe d'interposition (3) présente une face concave (32) et une face (35) délimitant la ou les arêtes aptes à venir en appui contre l'organe de liaison.

6. Système selon la revendication 5, **caractérisé en ce que** la face concave est une portion de face cylindrique dont un diamètre moyen est inférieur à un diamètre de l'organe de liaison.

7. Système selon la revendication 5 ou 6, **caractérisé en ce que** la face concave est délimitée par au moins deux arêtes aptes à réaliser l'appui contre l'organe de liaison.

8. Système selon l'une des revendications 1 à 7, **caractérisé en ce que** l'organe d'interposition (3) est monté en rotation sur le verrou.

9. système selon l'une des revendications 1 à 8, **caractérisé en ce qu'**il présente des moyens de retenue (33, 46) de l'organe d'interposition (3) directement sur le verrou.

10. Système selon la revendication 9, **caractérisé en ce que** les moyens de retenue (33,46) comportent une lèvre (46).

11. Système selon la revendication 10, **caractérisé en ce que** les moyens de retenue (33,46) comprennent au moins un ergot (33) apte à venir en prise avec la lèvre (46).

12. Système selon la revendication 11, **caractérisé en ce que** le verrou comporte la lèvre et l'organe d'interposition (3) comporte l'ergot.

13. Système selon la revendication 11, **caractérisé en ce que** le verrou comporte l'ergot et l'organe d'interposition (3) comporte la lèvre.

14. Système selon la revendication 1, **caractérisé en ce que** les moyens de retenue (33,46) comprennent un filetage apte à être retenu prisonnier par la lèvre.

15. Système selon l'une des revendications 1 à 14, **caractérisé en ce que** la lèvre fait partie d'une gorge.

16. Système selon l'une des revendications 1 à 15, **caractérisé en ce que** l'organe d'interposition (3) présente entre ses faces supérieure et inférieure une épaisseur (e) tel que lesdites faces sont sensiblement indéformables lors d'un serrage du système.

## Claims

1. Spinal osteosynthesis system comprising an anchoring member (1), a linking member (2), a lock (4), and an insert member (3) which latter member can be placed in an assembly position between the lock and the linking member when these members are accepted in the anchoring member and are arranged in such a way as not to protrude beyond the anchoring member in the assembly position, the linking member is a rod of circular section, **characterized in that** the insert member (3) exhibits at least one edge and **in that** the insert member is capable of bearing against the linking member only along the edge or edges.

2. System according to Claim 1, **characterized in that** the insert member (3) is designed to bear against the linking member along at least one generatrix of the linking member.

3. System according to Claim 1 or 2, **characterized in that** the insert member (3) is designed to bear against the linking member along two parallel generatrices of the linking member.

4. System according to one of Claims 1 to 3, **characterized in that** the insert member (3) exhibits a planar surface (31) perpendicular to an axis of revoloution of the lock and capable of bearing against the lock.

5. System according to one of Claims 1 to 4, **characterized in that** the insert member (3) has a concave face (32) and a face (35) delimiting the edge or edges capable of bearing against the linking member.

6. System according to Claim 5, **characterized in that** the concave face is a portion of a cylindrical face, a mean diameter of which is smaller than a diameter of the linking member.

7. System according to Claim 5 or 6, **characterized in that** the concave face is delimited by at least two edges capable of bearing against the linking member.

8. System according to one of Claims 1 to 7, **characterized in that** the insert member (3) is rotatably mounted on the lock.

9. System according to one of Claims 1 to 8, **characterized in that** it has retaining means (33, 46) for holding the insert member (3) directly on the lock.

10. System according to Claim 9, **characterized in that** the retaining means (33, 46) comprise a lip (46).

11. System according to Claim 10, **characterized in that** the retaining means (33, 46) comprise at least a peg (33) capable of engaging with the lip (46).

12. System according to Claim 11, **characterized in that** the lock comprises the lip and the insert member (3) comprises the peg.

13. System according to Claim 11, **characterized in that** the lock comprises the peg and the insert member (3) comprises the lip.

14. System according to Claim 1, **characterized in that** the retaining means (33, 46) comprise a screw thread that can be held captive by the lip.

15. System according to one of Claims 1 to 14, **characterized in that** the lip forms part of a groove.

16. System according to one of Claims 1 to 15, **characterized in that** the insert member (3) has, between its upper and lower faces, a thickness (e) such that the said faces are substantially non-deformable when the system is tightened.

## Patentansprüche

1. Wirbelosteosynthese-System, das ein Verankerungsorgan (1), ein Verbindungsorgan (2), ein Schließteil (4) und ein Zwischenorgan (3) umfaßt, das dazu geeignet ist, in Montageposition zwischen dem Schließteil und dem Verbindungsorgan angeordnet zu werden, wenn diese im Verankerungsorgan aufgenommen sind und so angeordnet sind, daß das Verankerungsorgan in Montageposition nicht überschritten wird, wobei das Verbindungsorgan eine Stange mit kreisförmigem Querschnitt ist, **dadurch gekennzeichnet, daß** das Zwischenorgan (3) mindestens eine Kante aufweist, und daß das Zwischenorgan dazu geeignet ist, eine Auflage auf dem Verbindungsorgan lediglich längs der Kante oder Kanten herzustellen.

2. System nach Anspruch 1, **dadurch gekennzeichnet, daß** das Zwischenorgan (3) dazu angeordnet ist, um auf dem Verbindungsorgan längs mindestens einer Erzeugenden des Verbindungsorgans zur Auflage zu kommem.

3. System nach Anspruch 1 oder 2, **dadurch gekennzeichnet, daß** das Zwischenorgan (3) dazu angeordnet ist, um auf dem Verbindungsorgan längs zweier Erzeugender des Verbindungsorgans zur Auflage zu kommen.

4. System nach einem der Ansprüche 1 bis 3, **dadurch gekennzeichnet, daß** das Zwischenorgan (3) eine ebene Fläche (31) aufweist, die senkrecht zu einer Drehachse des Schließteils verläuft und geeignet ist, zur Auflage am Sperrteil zu gelangen.

5. System nach einem der Ansprüche 1 bis 4, **dadurch gekennzeichnet, daß** das Zwischenorgan (3) eine konkave Fläche (32) und eine Fläche (35) aufweist, die die Kante oder Kanten begrenzen, die geeignet sind, auf dem Verbindungsorgan zur Auflage zu gelangen.

6. System nach Anspruch 5, **dadurch gekennzeichnet, daß** die konkave Fläche ein Teil einer zylindrischen Fläche ist, deren mittlerer Durchmesser kleiner ist als ein Durchmesser des Verbindungsorganes.

7. System nach Anspruch 5 oder 6, **dadurch gekennzeichnet, daß** die konkave Fläche durch mindestens zwei Kanten begrenzt ist, die geeignet sind, die Auflage auf dem Verbindungsorgan herzustellen.

8. System nach einem der Ansprüche 1 bis 7, **dadurch gekennzeichnet, daß** das Zwischenorgan (3) auf dem Schließteil drehbar angebracht ist.

9. System nach einem der Ansprüche 1 bis 8, **dadurch gekennzeichnet, daß** es Mittel zum Halten (33, 46) des Zwischenorgans (3) unmittelbar auf dem Schließteil aufweist.

10. System nach Anspruch 9, **dadurch gekennzeichnet, daß** die Mittel zum Halten (33, 46) eine Lippe (46) umfassen.

11. System nach Anspruch 10, **dadurch gekennzeichnet, daß** die Mittel zum Halten (33, 46) mindestens einen Nocken (33) umfassen, der geeignet ist, mit der Lippe (46) in Eingriff zu gelangen.

12. System nach Anspruch 11, **dadurch gekennzeichnet, daß** das Schließteil die Lippe umfaßt, und das Zwischenorgan (3) den Nocken umfaßt.

13. System nach Anspruch 11, **dadurch gekennzeichnet, daß** das Schließteil den Nocken umfaßt, und das Zwischenorgan (3) die Lippe umfaßt.

14. System nach Anspruch 1, **dadurch gekennzeichnet, daß** die Mittel zum Halten (33, 46) ein Gewinde umfassen, das geeignet ist, von der Lippe eingeschlossen zu werden.

15. System nach einem der Ansprüche 1 bis 14, **dadurch gekennzeichnet, daß** die Lippe den Teil einer Auskehlung bildet.

16. System nach einem der Ansprüche 1 bis 15, **dadurch gekennzeichnet, daß** das Zwischenorgan (3) zwischen seiner oberen und seiner unteren Fläche eine solche Dicke (e) aufweist, daß die genannten Flächen während des Festklemmens des Systems im wesentlichen unverformbar sind.
